# EUROPEAN PATENT APPLICATION

(11) **EP 4 707 297 A1**
(43) Date of publication of application: **11.03.2026**
(21) Application number: 24797479.3
(22) Date of filing: 26.04.2024
(51) Int. Cl.: C07K 14/705, C12N 15/62, C12N 5/074

(54) **RECOMBINANT EXTRACELLULAR VESICLES CONTAINING IMMUNOGLOBULIN FC REGION OR VARIANT THEREOF AND METHOD FOR ISOLATING EXTRACELLULAR VESICLES USING SAME**

(30) Priority: 26.04.2023 KR 20230054853
(71) Applicant: Inexoplat, Inc., Pohang-si, Gyeongsangbuk-do 37554 (KR); Handong Global University Industry-Academic Cooperation Foundation, Pohang-si, Gyeongsangbuk-do 37554 (KR)
(72) Inventor: KIM, Sung Hwan, Incheon 21986 (KR); KIM, Yoon Young, Incheon 22000 (KR); LEE, Jae Hwan, Incheon 21986 (KR); YU, Ho Sung, Incheon 22009 (KR); KO, Min Sung, Incheon 22009 (KR); LEE, Hyeon Ji, Incheon 21986 (KR); HONG, Eun Be, Yongin-si, Gyeonggi-do 16874 (KR); LEE, Jungmin, Pohang-si, Gyeongsangbuk-do 37669 (KR)
(74) Representative: HGF
(86) International application number: PCT/KR2024/005696
(87) International publication number: WO 2024/225825

(57) **Abstract**

The present invention relates to recombinant extracellular vesicles comprising an immunoglobulin Fc region or a variant thereof, and a method for isolating extracellular vesicles using the same. By utilizing the affinity between the immunoglobulin Fc region and specific protein ligands, extracellular vesicles can be isolated with high purity and efficiency. The extracellular vesicles can be isolated with high purity by the simple and straightforward method affinity chromatography, and thus is highly apt to be advantageously applicable in the diagnosis and treatment of various diseases.

## Description

### Technical Field

The present invention relates to recombinant extracellular vesicles comprising an immunoglobulin Fc region or a variant thereof, and a method for isolating extracellular vesicles using the same.

### Background Art

Extracellular vesicles are nanometer-sized vesicles composed of lipid bilayers, and comprise various types of physiologically active substances such as proteins, lipids, RNA (including microRNA and lncRNA) that exist inside the parent cell, and are known to play a very important role in the transmission of information between cells. In particular, extracellular vesicles isolated from stem cells are attracting attention as an alternative that can overcome various shortcomings of cell therapeutic agents because they have the therapeutic efficacy of stem cells while being cell-free.

Extracellular vesicles have the advantage of being safe because they do not induce immune responses and have a very low risk of tumorigenesis because they cannot self-replicate like cells. In addition, extracellular vesicles are much smaller than cells (10,000 to 20,000 nm in size), so they can be administered without the risk of embolism, a potential risk factor for cell therapeutic agents. Currently, attempts are being actively made to apply the extracellular vesicles to the treatment of various diseases.

Meanwhile, in order to apply the extracellular vesicles to therapeutic agents, it is crucial to isolate the extracellular vesicles with high purity. Current methods for isolating extracellular vesicles include ultracentrifugation, density gradient centrifugation, ultrafiltration, tangential flow filtration, size exclusion chromatography, ion exchange chromatography, immunoaffinity capture, microfluidics-based isolation, extracellular vesicle precipitation, total extracellular vesicle isolation kits, and polymer based precipitation, etc. (Tamas Baranyai et al., PLoS One. 2015; 10(12): e0145686). However, these methods have the disadvantages of relatively low purity and isolation efficiency of the extracellular vesicles being isolated, and being labor-intensive, requiring significant time and cost.

Therefore, there is a need for the development of a technology capable of isolating extracellular vesicles with high purity and efficiency within a short period of time, without expensive equipment or complex steps.

### Detailed Description of Invention

### Technical Problem

Accordingly, the present inventors prepared extracellular vesicles comprising an immunoglobulin Fc region and confirmed that the extracellular vesicles could be isolated with high purity and efficiency by utilizing the affinity of the Fc region with a protein ligand. Based on the above, the present inventors completed the present invention.

### Solution to Problem

In order to achieve the above object, in one aspect of the present invention, there is provided a fusion protein comprising an extracellular vesicle membrane protein and an immunoglobulin Fc region or a variant thereof.

In another aspect of the present invention, there is provided a polynucleotide encoding the fusion protein.

In another aspect of the present invention, there is provided a cell comprising the polynucleotide.

In another aspect of the present invention, there is provided a method for producing extracellular vesicles from the cell culture solution.

In another aspect of the present invention, there is provided an extracellular vesicle comprising an immunoglobulin Fc region or a variant thereof.

### Effects of Invention

Extracellular vesicles comprising an immunoglobulin Fc region or a variant thereof according to the present invention can be isolated with high purity and efficiency by utilizing the affinity between the immunoglobulin Fc region and specific protein ligands. The extracellular vesicles can be isolated with high purity by the simple and straightforward method affinity chromatography, and thus is highly apt to be advantageously applicable in the diagnosis and treatment of various diseases.

### Brief Description of Drawings

Figure 1a shows the results obtained by performing direct PCR with the hCD63 (Back) primer to confirm whether a mutated Fc gene was introduced into the induced pluripotent stem cells (iPSCs) according to the present invention. CTRL represents wild type iPSCs, and KI-iPSC represents iPSCs into which a mutated Fc gene was introduced.
Figure 1b shows the results obtained by extracting genomic DNA and performing PCR with the hCD63 (Back) primer to confirm whether a mutated Fc gene was introduced into the iPSCs according to the present invention. CTRL represents wild type iPSCs, and KI-iPSC represents iPSCs into which a mutated Fc gene was introduced.
Figure 2a shows the results obtained by performing sequencing with the hCD63 (Front_2) primer to confirm whether a mutated Fc gene was introduced into the iPSCs according to the present invention.
Figure 2b shows the results obtained by performing sequencing with the hCD63 (Full_2) primer to confirm whether a mutated Fc gene was introduced into the iPSCs according to the present invention.
Figure 2c shows the results obtained by performing sequencing with the hCD63 (Back) primer to confirm whether a mutated Fc gene was introduced into the iPSCs according to the present invention.
Figure 3a shows the results obtained by measuring the size and the particle number distribution by size of exosomes isolated from wild type iPSCs at each step of the exosome isolation method according to the present invention, using an NTA device. The X-axis represents particle size (nm), and the Y-axis represents the number of particles (particles/mL).
Figure 3b shows the results obtained by measuring the size and the particle number distribution by size of exosomes isolated from iPSCs into which a mutated Fc gene was introduced at each step of the exosome isolation method according to the present invention, using an NTA device. The X-axis represents particle size (nm), and the Y-axis represents the number of particles (particles/mL).
Figure 4a shows the results obtained by measuring the particle number of exosomes isolated from wild type iPSCs at each step of the exosome isolation method according to the present invention.
Figure 4b shows the results obtained by measuring the particle number of exosomes isolated from iPSCs into which a mutated Fc gene was introduced at each step of the exosome isolation method according to the present invention.
Figure 5 is a schematic diagram showing the method of inserting a mutated IgG Fc gene.
Figure 6 shows the results obtained by confirming whether a mutated IgG Fc gene was introduced into HEK293 cells using a T7E1 assay.
Figure 7 shows the results obtained by confirming the Fc expression in a HEK293 cell population into which a mutated IgG Fc gene was introduced using flow cytometry.
Figure 8 shows the results obtained by confirming a HEK293 single cell clone expressing a mutated IgG Fc gene using flow cytometry.
Figure 9 shows the results obtained by confirming the Fc expression of a HEK293 single cell clone according to the present invention using Western blot.
Figure 10 is a schematic diagram showing the process of purifying exosomes from the HEK293 cell culture solution according to the present invention.
Figure 11 shows the results obtained by comparing the expression of anti-IgG Fc, CD63, CD9, and CD81 in each fraction eluate obtained during the process of purifying the HEK293 cell culture solution according to the present invention.
Figure 12 shows the results obtained by comparing the presence or absence of calnexin in Elution 2 obtained during the process of purifying the HEK293 cell culture solution according to the present invention.
Figure 13 is a graph showing the level of Fc expression in each eluate obtained during the process of purifying the HEK293 cell culture solution according to the present invention.
Figure 14 shows the results obtained by confirming the presence or absence of Fc expression in exosomes obtained from the HEK293 cell culture solution.

### Mode for Carrying out the Invention

### Fusion protein comprising extracellular vesicle membrane protein and Fc region

In one aspect of the present invention, there is provided a fusion protein comprising an extracellular vesicle membrane protein and an immunoglobulin Fc region or a variant thereof.

As used herein, the term "extracellular vesicle (EV)" refers to a nano-sized particle naturally secreted by living cells, packaged in a lipid bilayer, and may be a concept that includes all vesicles (exosomes, microvesicles, multivesicles, and extracellular vesicle-like vesicles) that serve as information transfer mechanisms between cells and have a composition similar to that of extracellular vesicles.

As used herein, the term "membrane protein" refers to a protein embedded in or attached to the surface of a membrane formed by a lipid bilayer. Proteins embedded in the lipid bilayer are called integral membrane proteins, while proteins attached to the surface are called peripheral membrane proteins.

As used herein, the term "immunoglobulin (Ig)" refers to a glycoprotein molecule produced in an immune response to antigen stimulation. It primarily binds specifically to specific antigens in the blood and initiates an antigen-antibody reaction. It is also known as an antibody. It is produced by B lymphocytes and functions to eliminate antigens such as bacteria and viruses or to induce various immune functions by interacting with other components of the immune system.

As used herein, the term "Fc region (fragment crystallizable region)" refers to the C-terminal region of an immunoglobulin and one of the functional structural units comprising the CH2 and CH3 domains among the heavy chain constant regions. Fc does not have the ability to bind antigens, but exhibits binding to complement, etc., and has a consistent amino acid sequence.

The Fc region may be derived from IgG, IgA, IgD, IgM, IgE, or a combination thereof. Specifically, the Fc region may be derived from IgG.

The Fc region may be bound to the N-terminus or C-terminus of the extracellular vesicle membrane protein, or may be included within the extracellular vesicle membrane protein.

The extracellular vesicle membrane protein may be, but is not limited to, an endosome-associated protein (e.g., Alix, TSG101, Rab protein), a lipid raft-associated protein (e.g., glycosylphosphatidylinositol, flotillin), a lipid rich in cholesterol, sphingomyelin, and glycerophospholipid, a transmembrane protein (e.g., LAMP-1, CD13), a protein associated with membrane trafficking and fusion (e.g., LAMP-1, LAMP-2), an adhesion molecule (e.g., MFGE8, ICAM-1, CD58), an antigen-presenting protein, or a tetraspanin (e.g., CD63, CD81, CD9), and any protein constituting the extracellular vesicle membrane may be applied without limitation.

Specifically, the extracellular vesicle membrane protein may be CD63, CD81, CD9, or a combination thereof. CD63, CD81, and CD9 are tetraspanins, each having four alpha helices that penetrate the membrane and two extracellular domains. Specifically, the Fc region may be included within the extracellular domain of the extracellular vesicle membrane protein.

CD63, CD81, or CD9 may include a wild type, variant, isotype, or paralog thereof present in an animal, preferably a human.

The interior of CD63 may be located between the 32nd amino acid residue and the 53rd amino acid residue or between the 105th amino acid residue and the 209th amino acid residue in the amino acid sequence of SEQ ID NO: 1. This represents the amino acid sequence of the two extracellular domains constituting CD63. Specifically, the Fc region may be included in one or two extracellular domains of CD63.

The interior of CD81 may be located between the 34th amino acid residue and the 63rd amino acid residue or between the 112th amino acid residue and the 201st amino acid residue in the amino acid sequence of SEQ ID NO: 2. This represents the amino acid sequence of the two extracellular domains constituting CD81. Specifically, the Fc region may be included in one or two extracellular domains of CD81.

The interior of CD9 may be located between the 36th amino acid residue and the 55th amino acid residue or between the 113th amino acid residue and the 196th amino acid residue in the amino acid sequence of SEQ ID NO: 3. This represents the amino acid sequence of the two extracellular domains constituting CD9. Specifically, the Fc region may be included in one or two extracellular domains of CD9.

The Fc region may comprise CH2 and CH3 domains. The Fc region may have the amino acid sequence of SEQ ID NO: 4.

The Fc variant may have a modified CH2 domain. Specifically, it may have a partial substitution of an amino acid residue in the CH2 domain of the heavy chain constant region of an immunoglobulin. At this time, the Fc variant may have the amino acid sequence of SEQ ID NO: 5. The CH2 domain may have the amino acid sequence of SEQ ID NO: 6. At this time, the CH3 domain may have the amino acid sequence of SEQ ID NO: 7.

As used herein, the term "Fc variant" refers to a form in which a partial substitution of an amino acid residue in a full-length Fc or an Fc fragment occurs. In other words, the Fc variant may have an amino acid sequence different from that of a wild type Fc or a fragment thereof. However, the Fc variant may have activity equivalent to or similar to that of a wild type Fc.

Specifically, the Fc variant may have some amino acid substitutions to eliminate immunological functions, such as ADCC (antibody-dependent cellular cytotoxicity) and CDC (complement-dependent cytotoxicity), through binding to Fc receptors or complement in the Fc region. As a specific example, the 18th amino acid residue, glutamic acid, in the amino acid sequence of SEQ ID NO: 4 may be substituted with another amino acid residue. In addition, as a specific example, glutamic acid may be substituted with proline (SEQ ID NO: 5).

In another aspect of the present invention, there is provided a polynucleotide encoding the fusion protein.

In another aspect of the present invention, there is provided a cell comprising the polynucleotide. The cell may be a cell line or stem cell for protein production.

Specifically, the cell line for protein production may be any one selected from the group consisting of CHO cells, F2N cells, COS cells, BHK cells, Bowes melanoma cells, HeLa cells, 911 cells, AT1080 cells, A549 cells, SP2/0 cells, NSO cells, HT-1080 cells, PERC.6 cells, HEK 293 cells, and HEK293T cells, but is not limited thereto. Specifically, the cell line for protein production may be HEK 293 cells.

In addition, the stem cell may be any one selected from the group consisting of a mesenchymal stem cell, a hematopoietic stem cell, a neural stem cell, an induced pluripotent stem cell, a pluripotent stem cell, and an embryonic stem cell, but is not limited thereto.

In addition, the mesenchymal stem cell may be an induced pluripotent stem cell-derived mesenchymal stem cell or an embryonic stem cell-derived mesenchymal stem cell, but is not limited thereto.

In one embodiment, a plasmid-type DNA encoding a fusion protein comprising an Fc variant in the extracellular domain of CD63 may be introduced into induced pluripotent stem cells.

In another aspect of the present invention, there is provided a method for producing extracellular vesicles comprising an Fc region or a variant thereof, comprising: isolating extracellular vesicles from the cell culture solution.

### Recombinant extracellular vesicle comprising immunoglobulin Fc region

In another aspect of the present invention, there is provided a recombinant extracellular vesicle comprising an immunoglobulin Fc region or a variant thereof.

As used herein, the term "recombinant extracellular vesicle" is a concept opposite to natural extracellular vesicles produced naturally, and refers to an artificial extracellular vesicle produced by recombinant methods that comprise the exogenous protein within or on its membrane surface by being produced in a host cell transformed to produce an exogenous protein expressed by the exogenous polynucleotide by introducing an exogenous polynucleotide into the host cell.

In one embodiment, the recombinant extracellular vesicle may be produced in a host cell transformed to express an Fc region by introducing an exogenous polynucleotide encoding an Fc region, thereby comprising the Fc region within the extracellular vesicle or on its outer surface.

The host cell is a cell capable of producing extracellular vesicles. It may be a cell line, B cell, T cell, natural killer (NK) cell, macrophage, dendritic cell, fibroblast, or stem cell, but is not limited thereto. In one embodiment, it may be an induced pluripotent stem cell.

The Fc region may be present on the outer surface of the extracellular vesicle.

The Fc region may be bound to the extracellular vesicle membrane protein.

The extracellular vesicle membrane protein may be, but is not limited to, an endosome-associated protein (e.g., Alix, TSG101, Rab protein), a lipid raft-associated protein (e.g., glycosylphosphatidylinositol, flotillin), a lipid rich in cholesterol, sphingomyelin, and glycerophospholipid, a transmembrane protein (e.g., LAMP-1, CD13), a protein associated with membrane trafficking and fusion (e.g., LAMP-1, LAMP-2), an adhesion molecule (e.g., MFGE8, ICAM-1, CD58), an antigen-presenting protein, or a tetraspanin (e.g., CD63, CD81, CD9), and any protein constituting the extracellular vesicle membrane may be applied without limitation.

Specifically, the extracellular vesicle membrane protein may be any one selected from the group consisting of Alix, Tsg101, Rab protein, ICAM, CD106, CD82, CD58, CD53, CD37, CD13, MFGE8, IGSF8, PTGFRN, MARCKS, MARKCSL1, BASP1, LAMP-1, LAMP-2, CD9, CD81, CD63, and a combination thereof. In one embodiment, the extracellular vesicle membrane protein may be CD63.

If the extracellular vesicle membrane protein is a peripheral membrane protein, the Fc region may be bound to the N-terminus or C-terminus of the extracellular vesicle membrane protein, or may be included within the extracellular vesicle membrane protein.

If the extracellular vesicle membrane protein is an integral membrane protein, the Fc region may be bound to at least one selected from the group consisting of the N-terminus, C-terminus, and extracellular domain present outside the cell of the extracellular vesicle membrane protein. Specifically, when the extracellular vesicle membrane protein is an integral membrane protein, the Fc region may be bound to a domain present outside the cell of the extracellular vesicle membrane protein, rather than a transmembrane domain or an intracellular domain.

The Fc region may comprise CH2 and CH3 domains.

The Fc variant may have a modified CH2 domain.

The Fc variant may be as described above, but is not limited thereto.

The Fc may be derived from IgG, IgA, IgD, IgM, IgE, or a combination thereof.

### Method for isolating extracellular vesicles

In another aspect of the present invention, there is provided a method for isolating extracellular vesicles, comprising: a) culturing cells comprising a polynucleotide encoding an immunoglobulin Fc region or a variant thereof; b) loading a cell culture solution onto a column comprising a protein ligand; and c) loading an eluate onto the column to isolate the recombinant extracellular vesicles included in the cell culture solution.

The protein ligand may be any protein that exhibits affinity for an immunoglobulin Fc region or a variant thereof. Specifically, it may be protein A, protein G, or protein A/G.

Protein A is a protein present in the cell wall of *Staphylococcus aureus* and is known to bind to the Fc region and the Fab region of mammalian immunoglobulins. While it has strong affinity for the Fc region, it readily dissociates under acidic conditions (pH 3.0).

The protein A ligand may be a full-length domain protein A ligand. That is, the protein A ligand may comprise all full-length domains (E, D, A, B, and C domains). All domains of the protein A ligand are capable of binding to the Fc region.

The protein G is a protein isolated from the cell wall of group G streptococci and is known to bind to the Fc region and the Fab region of mammalian immunoglobulins.

The protein G ligand may be a full-length domain protein A ligand. That is, the protein G ligand may comprise all full-length domains (E, A1, B1, A2, B2, A3, S, C1, D1, C2, W, and M domains). The C1 and C2 domains of the protein G ligand are capable of binding to the Fc region.

The protein A/G is a recombinant fusion protein in which the IgG binding domains of protein A and protein G are combined.

The eluate may be at a specific pH condition (e.g., pH 3.0) that allows for the isolation of the protein ligand and extracellular vesicles.

The method may further comprise neutralizing the isolated extracellular vesicles with a neutralizing buffer.

Duplicate contents are omitted in consideration of the complexity of the present specification, and terms not otherwise defined in the present specification have meanings commonly used in the art to which the present invention pertains.

Hereinafter, the present invention will be described in more detail by way of the following examples. However, the following examples are only for illustrating the present invention, and the scope of the present invention is not limited only to these examples.

### Preparation Example 1. Culture of induced pluripotent stem cells

Human fibroblast-derived induced pluripotent stem cell (iPSC) line (ATCC, ACS-1019) was cultured in TeSR-E8 ACF 2-Comp Medium (E8) (Stem cell technologies) supplemented with 10 ng/mL basic fibroblast growth factor (bFGF) at 37 °C and 5% CO₂ conditions. The cell culture dish was coated with vitronectin.

### Example 1. Introduction of mutated IgG Fc gene into iPSCs via CRISPR-Cas9

For the mutated IgG Fc region to be introduced into iPSCs, the 'APELLGGP' amino acid sequence in the CH2 protein domain, which binds to the Fc receptor in the wild type IgG Fc region, was modified to 'APPLLGGP.' Specifically, glutamic acid (E) was substituted with proline (P). This mutated IgG Fc region was obtained through gene synthesis by Thermofisher Scientific's GeneArt Service. The DNA nucleotide sequence was codon-optimized to encode a protein having the amino acid sequence of SEQ ID NO: 5, and this was used as the donor DNA in plasmid form.

After 6-7 days of subculturing the iPSCs from Preparation Example 1, all cells were dissociated into single cells. Using the Neon transfection system (Thermofisher Scientific), 6 µg of Cas9 Nuclease (New England Biolabs), 5 µg of guide RNA (Synthego), and 1.1 µg of donor DNA plasmid were inserted into 4 x 10⁵ cells by electroporation. Electroporation conditions were 1050 V, 30 ms, and 2 pulses. The base sequences of the guide RNA and donor DNA are listed in Table 1 below.

**[Table 1]**

| SEQ ID NO | Name | Nucleotide sequence of target site (5'-> 3') | Modifications |
|---|---|---|---|
| 8 | target of guide RNA | CCT GAG TCA GAC CAT AAT CCA GG | 2'-O-methyl 3' phosphorothioate modifications at the first and last three nucleotides |

**[Table 2]**

| SEQ ID NO | Donor DNA | Nucleotide sequence (5'-> 3') |
|---|---|---|
| 9 | Left homology arm | TGGGTGTGCAGCAACCCCTACCCCCATCCCTCCTGGCCCTTCCTG TCTGGTGACCCAACCACTTGTGATATGGGGCAGTGGGGCAACCA GCCAACCGCCTGATGCTATCGCCATGGGGCTTCCCTTCGCTCTCC CTTCTCCCCTCTCTCCCTCCCCAACCCTCTCATCCCTGTCCAGCCC CTCTCCCCCTTTCCCTCCTCCCAGCGCTTGGGGGTGAGGAAGGGC TCTGCCCACTTCAATTTGCTCCTGCCCTGGGCTCCTGGCTTGAGA GGCTACTGTTTGTCTAGGGAGGGACACATCACTCTTGCTCCCTTT TCCTCTTCCCTTCTCCTGGCCTAACTGGCCAGAGGAACCTTAGTC ACAGCTGATGCGCGCGTGGGATGTGCACTGGAGGATAGTGAGGG GGCAGTTCTGGGAGAGAATTGAGCTGGGAGCCCTGGAGGCCTGT AGAAAGCATTATTCTAGGTCAGAAATACGTGTCTTGAGGTCAAGG CTTTCTGGGAGGTTTTTGAATTTGGTCATGTCCCTGATTAAAGAGC CGGGAGGGGATCAGGGGAGGTAGGGGCAGGCAAGGATGGGTCA |
| | | TCCCCCTACCCCACAGCCAAAGAAATTCCCTAGAGGTGTGAGAAT CTCTCTTCCGGATGGGGTCAAGTCTGTGTCCTAGGTGTACCCCCT GCCCTCCACCATGGAGGGCCACTGTGTTGTGGATATGGCCTAATC CTCACCTGATTTTCTCTTAGGCCTGTGCAGTGGGACTGATTGCCG TGGGTGTCGGGGCACAGCTTGTCCTGAGTCAGACCATA |
| 10 | Fc region | GAGCCCAAGTCCTGCGACAAGACCCACACCTGCCCCCCCTGCCC CGCCCCCCCACTCCTCGGCGGCCCCTCCGTCTTCCTCTTCCCCCC CAAGCCCAAGGACACCCTCATGATCTCCCGCACCCCCGAGGTCA CCTGCGTCGTCGTCGACGTCTCCCACGAGGACCCCGAGGTCAAG TTCAACTGGTACGTCGACGGCGTCGAGGTCCACAACGCCAAGAC CAAGCCCCGCGAGGAGCAGTACAACTCCACCTACCGCGTCGTCT CCGTCCTCACCGTCCTCCACCAGGACTGGCTCAACGGCAAGGAG TACAAGTGCAAGGTCTCCAACAAGGCCCTCCCCGCCCCCATCGA GAAGACCATCTCCAAGGCCAAGGGCCAGCCCCGCGAGCCCCAGG TCTACACCCTCCCCCCCTCCCGCGACGAGCTCACCAAGAACCAG GTCTCCCTCACCTGCCTCGTCAAGGGCTTCTACCCCTCCGACATC GCCGTCGAGTGGGAGTCCAACGGCCAGCCCGAGAACAACTACA AGACCACCCCCCCCGTCCTCGACTCCGACGGCTCCTTCTTCCTCT ACTCCAAGCTCACCGTCGACAAGTCCCGCTGGCAGCAGGGCAAC GTCTTCTCCTGCTCCGTCATGCACGAGGCCCTCCACAACCACTAC ACCCAGAAGTCCCTCTCCCTCTCCCCCGGCAAG |
| 11 | Right homology arm | ATCCAAGGAGCTACCCCTGGCTCTCTGTTGCCAGTGGTCATCATC GCAGTGGGTGTCTTCCTCTTCCTGGTGGCTTTTGTGGGCTGCTGC GGGGCCTGCAAGGAGAACTATTGTCTTATGATCACGGTGAGTTGG GGGCGGGACCAACATGGGCGGTCTGGGACTGCCAGAGCAGCTTG GTTCGGGCTCAGGAAGGTGGGTGAGGTGGGTGGGTGAGGAAGA CCTTTGTGAGTGTCCAGGGACCGGCTGTGCCAAAACGGGCCCCT GTAATGCATAGACTCCAAACTTGACTTCTGTCCTTTGCTCCTGCAG TTTGCCATCTTTCTGTCTCTTATCATGTTGGTGGAGGTGGCCGCAG CCATTGCTGGCTATGTGTTTAGAGATAAGGTAAGCAGGGAATAAT GGGAAGGGCCTGCCTCAGCCGGGTCTGGGACCCTGGTGCTTGAA TTCTGACTGCAACAGTGCTCCTCCGTACTCTCAGGTGATGTCAGA GTTTAATAACAACTTCCGGCAGCAGATGGAGAATTACCCGAAAAA CAACCACACTGCTTCGATCCTGGACAGGATGCAGGCAGATGTGA GTGGGGTTGCTGGGAGCAGGGGTGGTGGGAATAGAGAATTCTCA TCTCCAAGGCTGGGTGTGGTGACTCACGCCTGTAATCTCAGCACT TTTGAAGGCCGAGGTGGGCAGATCACCTGAGGTCAGAAGTTCAA GACTAGCCTGGCCAACGTGGTGAAACCCTGTCTCTACTAAAAATA |
| | | CAAAAATTAGCCAGGTGTGGTGGTGAGTGCCTGTAATCCCA |

The iPSCs that was subjected to electroporation were cultured in E8 medium supplemented with 10 µM Y27632 and 10 ng/mL bFGF for 48 hours, and then the medium was replaced with E8 medium supplemented with 10 ng/mL bFGF. Ten to 13 days after electroporation, colonies formed by single cells were isolated through colony picking and cultured in a 24-well plate.

### Experimental Example 1. Confirmation of whether mutated IgG Fc gene was introduced into iPSCs

### Experimental Example 1.1. Performance of Direct polymerase chain reaction

In order to confirm the gene introduction results using direct polymerase chain reaction (direct PCR), a portion of each iPSC colony isolated in Example 1 was removed and centrifuged at 5,000 rpm and 4 °C for 3 minutes to precipitate the cells. The supernatant was removed, and then the precipitate was washed with PBS (Welgene), and 7.5 mM Tris solution (pH 8.8) was added. 20 ng/mL Proteinase K was added to the cell suspension, and then the mixture was reacted at 55 °C for 1 hour and then reacted at 95 °C for an additional 10 minutes to obtain a cell lysate.

Next, the cell lysate was mixed with GoTaq^{®} G2 Green Master Mix (Promega) and primers, and direct PCR using a touchdown type was performed at 48 °C to 60 °C. The PCR products were electrophoresed on a 0.7% agarose gel at 100 V for 35 minutes.

The primers used in the direct PCR are listed in Table 3 below.

**[Table 3]**

| Gene | Primer | | SEQ ID NO | Annealing Temperature(°C) | Size (bp) (WT) | Size(bp) (KI) |
|---|---|---|---|---|---|---|
| hCD63 (Back) | Forward | AAC AAC TAC AAG ACC ACC C | 12 | 55.2 | - | 1536 |
| | Reverse | GAC AAA AGC ACC ATC AGA AAC | 13 | 57.4 | | |
| hCD63 (Full_2) | Forward | ACC ATA GAG CCC AAG TCC TG | 14 | 60.5 | - | 742 |
| | Reverse | TGA CCA CTG GCA ACA GAG AG | 15 | 60.5 | | |
| hCD63 (Front_2) | Forward | AGG GGA TCA GGG GAG GTA G | 16 | 61.7 | - | 706 |
| | Reverse | AGG GAG ACC TGG TTC TTG GT | 17 | 60.5 | | |

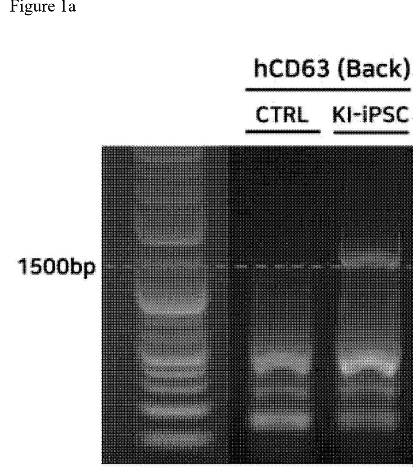

As a result, as shown in Figure 1a, it was confirmed that a product with a size of 1,500 bp was present in the direct PCR product of the iPSC (KI-iPSC) of Example 1 into which the mutated IgG Fc gene was introduced, but was not present in the direct PCR product of the iPSC of Preparation Example 1. Given this, it was confirmed that the mutated IgG Fc gene was introduced into the iPSC of Example 1.

In addition, genomic DNA was extracted from the obtained cell lysate and subjected to direct polymerase chain reaction. Specifically, 1% SDS was used to extract genomic DNA from each iPSC colony isolated in Example 1. The cells were lysed, and then protenase K was added, and the mixture was reacted at 55 °C for 4 hours. The genomic DNA was recovered using sodium chloride and ethanol, and then vortexed to break the genomic DNA into short fragments. Thereafter, GoTaq^{®} G2 Green Master Mix (Promega) and primers were mixed, and direct PCR was performed in a touchdown format at 48 °C to 60 °C. The PCR products were subjected to electrophoresis on a 0.7% agarose gel at 100 V for 35 minutes. The primers used are listed in Table 3 above.

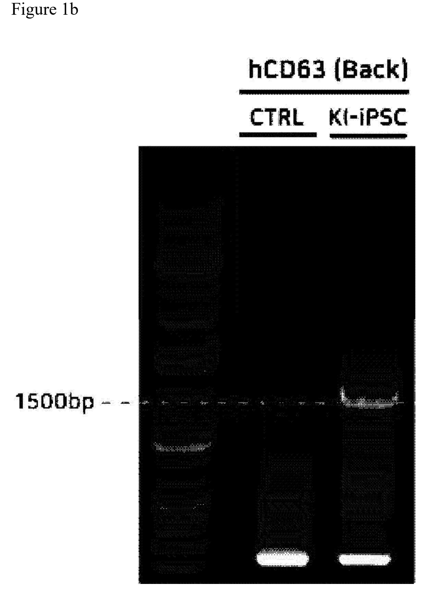

As a result, as shown in Figure 1b, it was confirmed that a product with a size of 1,500 bp was present in the product of direct PCR that was performed after extracting genomic DNA from the iPSC (KI-iPSC) of Example 1 into which the mutated IgG Fc gene was introduced, but was not present in the direct PCR product of the iPSC of Preparation Example 1. Given this, it was confirmed that the mutated IgG Fc gene was introduced into the iPSC of Example 1.

### Experimental Example 1.2. Sequencing analysis

In order to precisely determine the presence of gene introduction, the cell lysate obtained in Experimental Example 1.1 and the PCR product obtained using the primer set were subjected to sequencing analysis by Macrogen. The primers used for sequencing analysis are listed in Table 3 above.

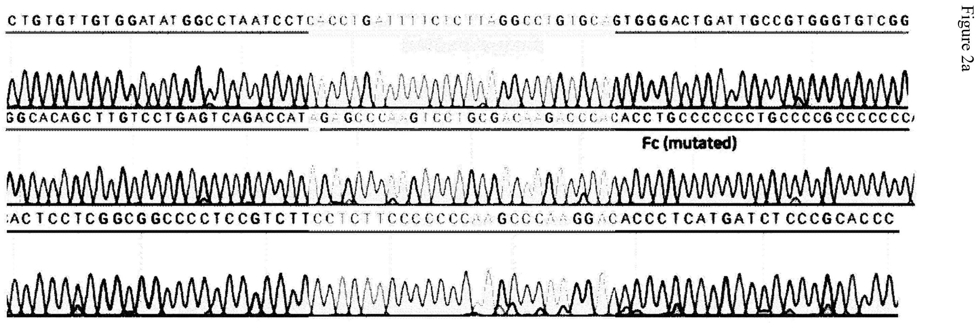

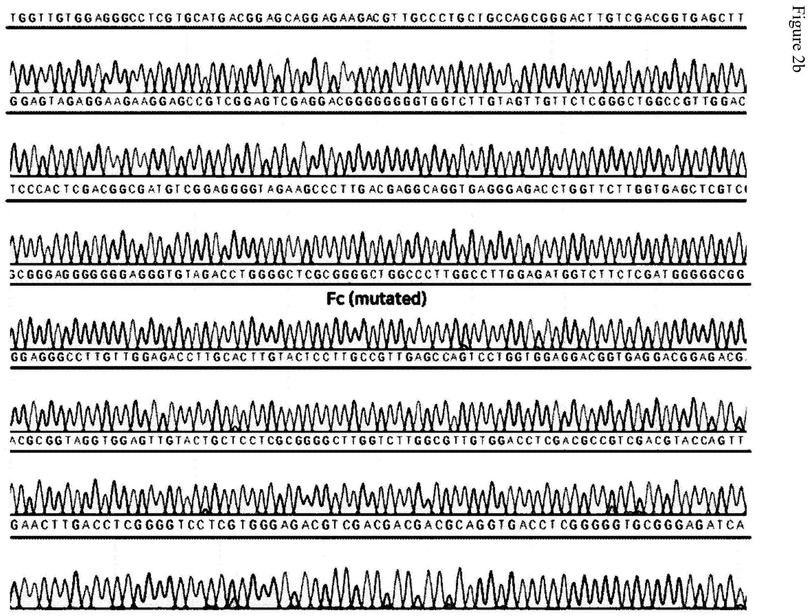

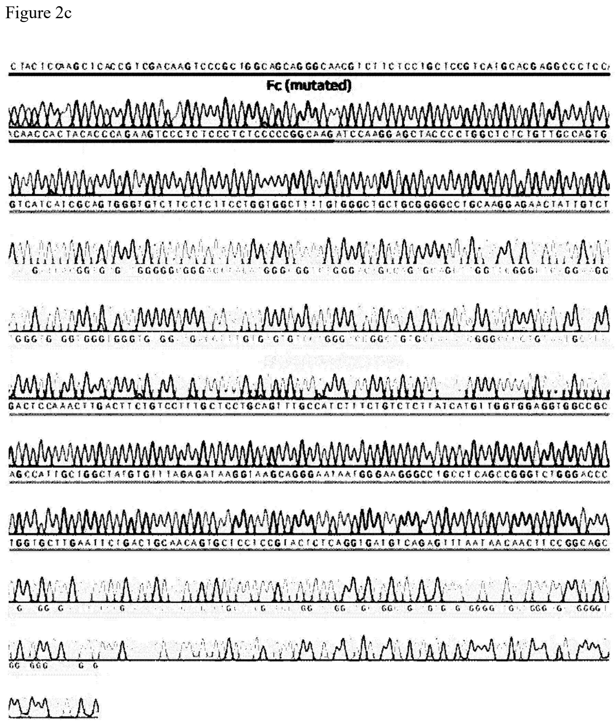

As a result, as shown in Figures 2a to 2c, it was confirmed that the mutated IgG Fc gene was introduced into the iPSC of Example 1.

iPSC colonies (knockin iPSCs; KI-iPSCs) confirmed to have the mutated IgG Fc gene introduced through direct PCR and sequencing analysis were isolated and expanded in a 6-well plate.

### Example 2. Isolation of exosomes from iPSCs (KI-iPSCs) into which the mutated IgG Fc gene was introduced

When the KI-iPSCs identified in Experimental Example 1.2 reached 70% to 80% of the culture dish surface area, the cell culture solution was harvested and centrifuged at 3,000 xg at 4 °C for 20 minutes to remove cells and large debris. The supernatant obtained from the centrifugation was filtered through a 0.2 µm Minisart^{™} NML syringe filter (Sartorius) to remove particles larger than 200 nm. Thereafter, the filtered supernatant was passed through a column equipped with Protein A resin and washed twice with 30 mL of PBS. Next, the exosomes were eluted with 2 mL of 0.1 M Glycine (pH 3.0) (elution buffer), and the eluate was neutralized with 2 mL of 1 M Tris-HCl (pH 8.8) (neutralization buffer).

### Comparative Example 1. Isolation of exosomes from iPSCs

The exosomes were isolated from the iPSCs of Preparation Example 1 in the same manner as described in Example 2.

### Experimental Example 2. Analysis of properties of exosomes isolated from iPSCs into which mutated IgG Fc gene was introduced

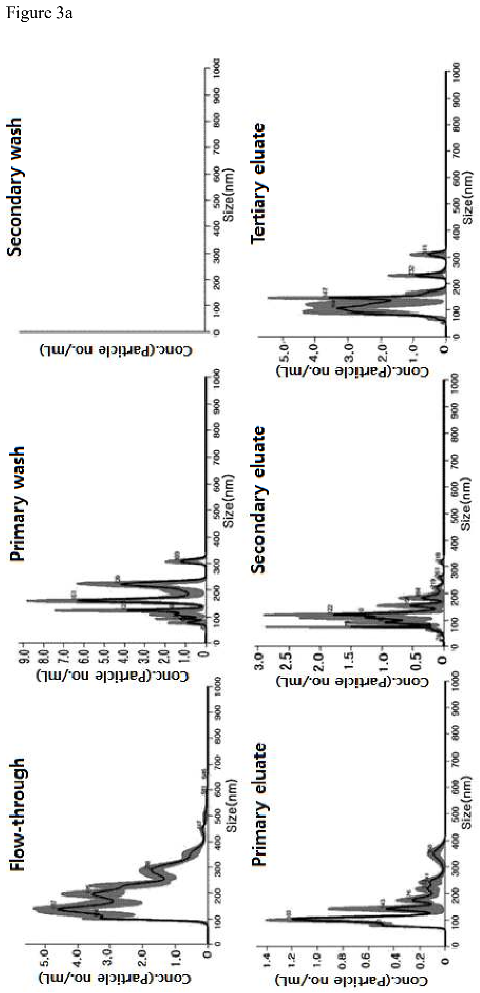

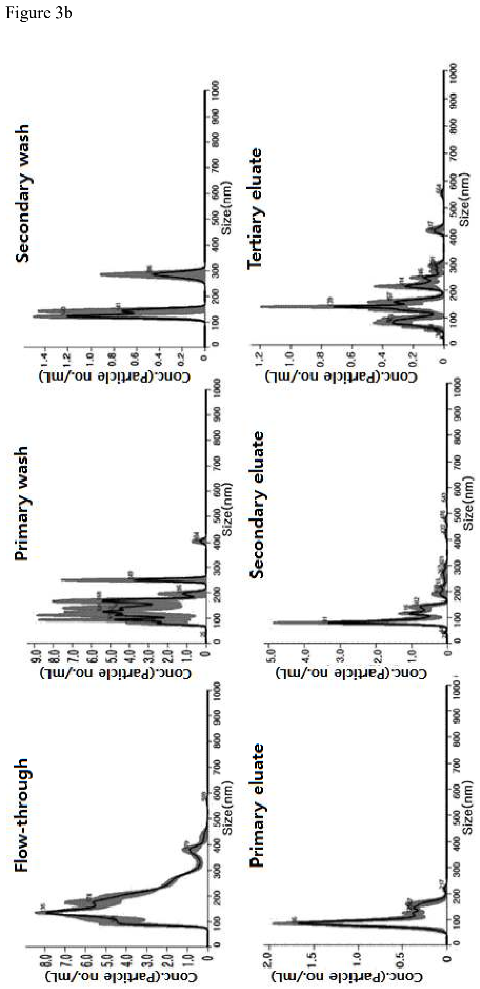

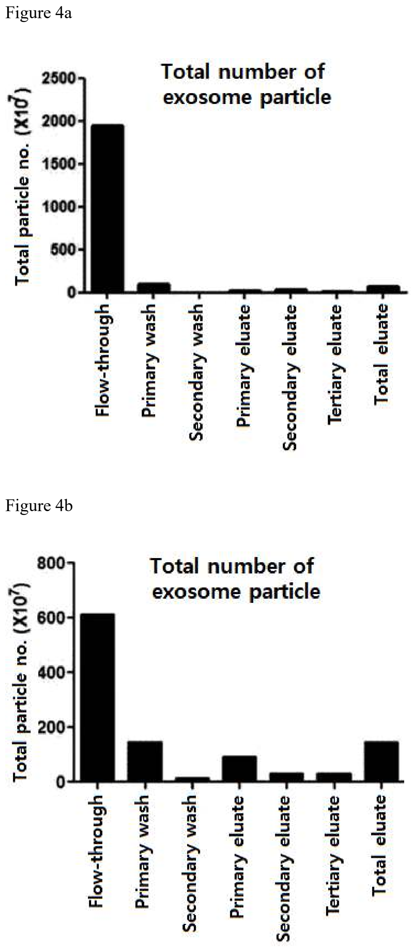

The size and the distribution by size of exosomes isolated from the iPSCs of Comparative Example 1 and exosomes isolated from the KI-iPSCs of Example 2 were analyzed using nanoparticle tracking analysis (NTA) (NS300, Malvern Panalytical). Specifically, the solution in which the cell culture solution was passed through the Protein A resin column (flow through), the solution in which PBS was passed through the Protein A resin column (1^{st} washing), the solution in which new PBS was passed through the column after the 1^{st} washing (2^{nd} washing), and the solution in which 2 mL of new elution buffer was passed through the column again after the 2^{nd} washing. Thereafter, a solution was obtained by mixing 2 mL of neutralization buffer with the solution that was passed through the column after reacting for 10 minutes at room temperature (1^{st} elute), a solution was obtained by mixing 2 mL of neutralization buffer with the solution that was passed through the column after obtaining the 1^{st} elute, passing a new 2 mL of elution buffer through the column again, and reacting for 20 minutes at room temperature (2^{nd} elute), and a solution was obtained by mixing 2 mL of neutralization buffer with the solution that was passed through the column after obtaining the 2^{nd} elute, passing a new 2 mL of elution buffer through the column again, and reacting for 30 minutes at room temperature (3^{rd} elute), and these were analyzed using an NTA device, and the results are shown in Figures 3a and 3b. In addition, the particle number of exosomes in each solution measured through the NTA device was summarized and graphed, and the results are shown in Figures 4a and 4b.

As shown in Figures 3a and 3b, the initial culture solution and the flow through included exosomes of various sizes and impurities. However, it was confirmed that by filtering the culture solution through a column, exosomes with sizes of 50 nm to 200 nm could be specifically eluted from the initial elute. In addition, in the case of the culture solution obtained in Comparative Example 1, since it was a general exosome into which the Fc gene was not introduced, almost all of the exosomes disappeared during the purification process. However, in the case of the culture solution obtained from iPSCs into which the mutated IgG Fc gene was introduced, it was confirmed through Figures 4a and 4b that a sufficient number of exosomes remained in the elute during the purification process.

Considering this together with the results in Figure 3a, this suggests that exosomes comprising the mutated IgG Fc are present within the KI-iPSC culture solution into which the mutated IgG Fc gene was introduced, and that these exosomes can be effectively isolated using a column comprising a protein ligand capable of binding to the mutated IgG Fc.

### Example 3. Introduction of IgG Fc gene into HEK293 cells via CRISPR-Cas9

Human IgG Fc protein knock-in (KI) cells were produced by introducing a donor gene together with the CRISPR/Cas9 system into HEK293 (human embryonic kidney 293) cells. The HEK293 cells were cultured in DMEM High glucose (Welgene, LM001-05) supplemented with 10% fetal bovine serum (FBS) and 1% penicillin-streptomycin. The cells were cultured in a 5% CO₂ incubator at 37 °C. The KI donor used for IgG Fc gene introduction was the same plasmid (Table 4) used for IgG Fc gene introduction into iPSCs in Example 1.1 above. The CRISPR/Cas9 system and the IgG Fc KI donor plasmid were introduced using lipofectamine.

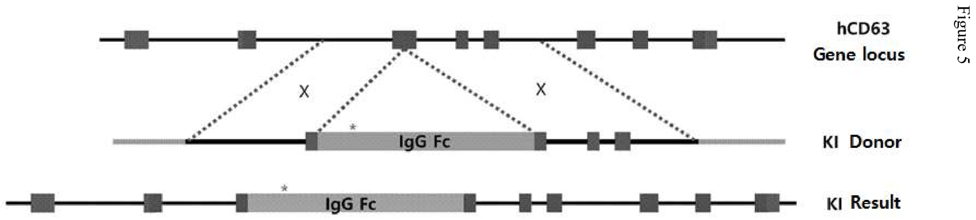

Figure 5 is a schematic diagram of gene insertion within a cell.

### Experimental Example 3. Confirmation of whether IgG Fc gene was introduced into HEK293 cells

Whether the desired genomic locus was cleaved by the introduced CRISPR/Cas9 system was confirmed using a T7E1 assay after amplifying the gDNA of the locus by PCR.

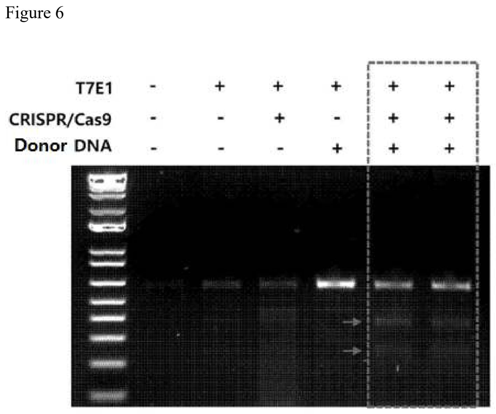

As a result, as shown in Figure 6, it was confirmed that a double strand break (DSB) occurred at the intended location of the CD63 genomic locus in cells delivered with the CRISPR/Cas9 system.

The primer sequences used for PCR amplification are listed in Table 4.

**[Table 4]**

| Primer | | SEQ ID NO |
|---|---|---|
| Forward | tggaggcctgtagaaagcat | 18 |
| Reverse | ccctggacactcacaaaggt | 19 |

### Experimental Example 4. Confirmation of Fc expression in HEK293 cell population into which IgG Fc gene was introduced

The presence of Fc-expressing cells in the HEK293 cells of Example 3 was confirmed by flow cytometry using PE Goat anti-human IgG FC (eBioScience, #2361125).

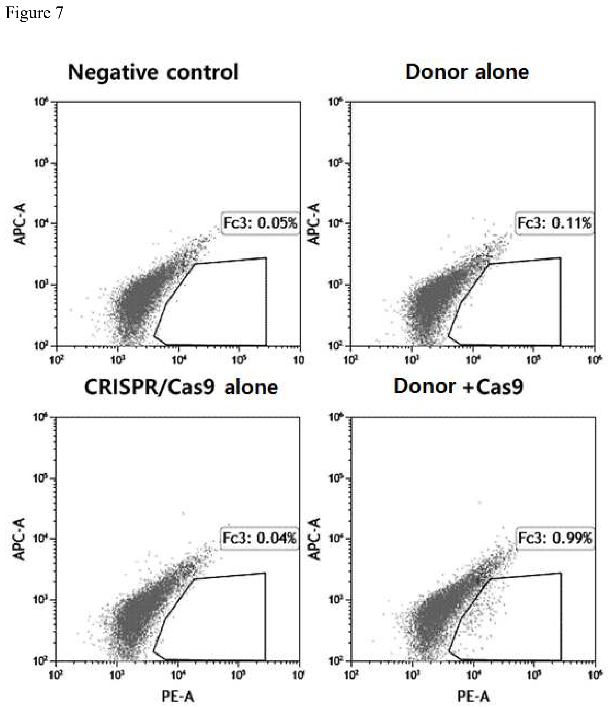

As a result, as shown in Figure 7, it was confirmed that only a small percentage of cells (0.99%) expressed Fc in the cell population transfected with the KI donor and the CRISPR/Cas9 system. Given this, it was confirmed that the IgG Fc gene was successfully knocked-in (KI) at a genomic locus in some HEK293 cells.

### Experimental Example 5. Obtaining HEK293 single cell clone into which IgG Fc gene was introduced

In order to obtain only Fc knock-in (KI) cells as single clones, the cells were dissociated and then multiple single cell clones were obtained through limiting dilution. Thereafter, the cell clones expressing Fc were selected using flow cytometry and managed separately.

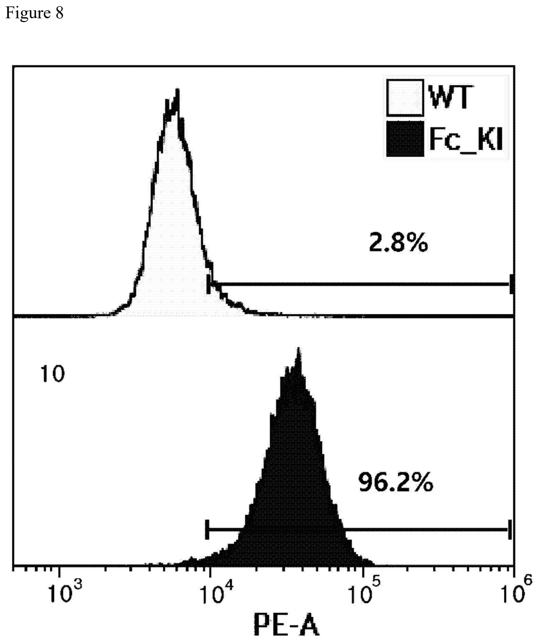

As a result, as shown in Figure 8, the presence or absence of Fc expression in the isolated single cell clones was confirmed through flow cytometry. It was confirmed that the expressing cells were purely isolated.

### Experimental Example 6. Analysis of Fc expression in HEK293 single cell clone into which IgG Fc gene was introduced

Western blotting was used to confirm the expression of Fc protein fused to CD63 in Fc knock-in (KI) single cell clones. Wild-type (WT) clones and IgG Fc KI single cell clones were washed with PBS and then resuspended in RIPA buffer containing proteinase inhibitors to lyse the cells. Thereafter, the concentration of cell lysate was quantified using the BCA assay, and then 4X Laemmli buffer was added to the same amount of cell lysate, denatured at 95 °C for 10 minutes, and electrophoresed on 10% SDS-PAGE gel. After electrophoresis, the eluate was transferred to PVDF membranes and blocked in 5% skin milk.

After blocking, the eluate was washed with TBS-T washing buffer. Thereafter, the eluate was incubated with target protein-specific antibodies (anti-IgG Fc, CD63, and GAPDH) and corresponding secondary antibodies. The eluate was then reacted with Supersignal West Pico Plus solution (Thermo Scientific, #34580) and analyzed using an iBright 1500 (Thermo Scientific) instrument. GAPDH was used as a control group to confirm that equal amounts of protein were loaded.

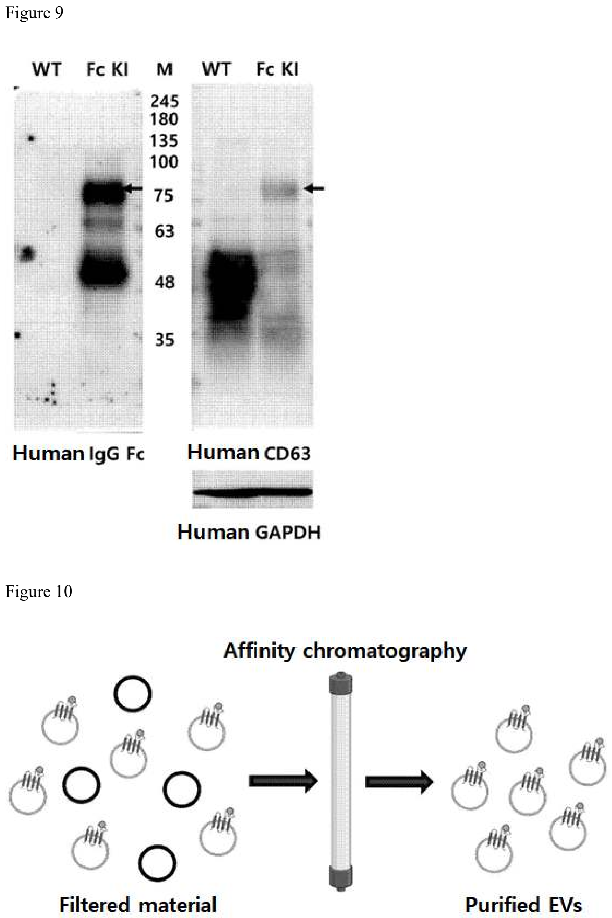

As a result, as shown in Figure 9, when Western blotting was performed with an Fc-specific antibody, it was confirmed that a band that was not detected in WT HEK293 cells was detected in KI cells. Specifically, a CD63-Fc fusion protein-specific band was observed in the range of 60 kDa to 95 kDa. Similarly, when Western blotting was performed with a CD63 antibody, it was confirmed that an endogenous CD63 band was detected in the range of 30 kDa to 65 kDa in WT HEK293 cells, whereas a band was detected between 60 kDa and 95 kDa in Fc-KI cells. Given this, it was confirmed that a CD63-Fc fusion protein, in which Fc is fused to the terminal of CD63, was expressed in the cells.

### Example 4. Isolation of exosomes from HEK293 cells into which IgG Fc gene was introduced

The culture solution was purified to remove cells and large debris, leaving only exosomes and impurities of similar size to exosomes.

Thereafter, impurities other than exosomes were removed by flow-through by passing through an affinity chromatography column that selectively binds to the Fc region expressed on the exosome surface. In order to elute the exosomes bound to the column, a pH-based elution method was utilized, and the eluate was collected into multiple fractions depending on the pH and eluate volume.

Figure 10 is a schematic diagram showing the process of purifying the culture solution.

### Experimental Example 7. Confirmation of exosome-specific marker expression in isolated exosomes

The concentrated RIPA buffer was added to each fraction eluate obtained in Example 4 to lyse the material in the eluate. Thereafter, the protein concentration in the eluate was quantified using the BCA assay, and then 4X Laemmli buffer was added to 25 µg of protein, denatured at 95 °C for 10 minutes, and electrophoresed on 10% SDS-PAGE gel. After electrophoresis, the eluate was transferred to PVDF membranes and blocked in 5% skin milk. After blocking, the eluate was washed with TBS-T washing buffer. Thereafter, the eluate was incubated with target protein-specific antibodies (anti-IgG Fc, CD63, CD9, and CD81) and corresponding secondary antibodies. The eluate was then reacted with Supersignal West Pico Plus solution (Thermo Scientific, #34580) and analyzed using an iBright 1500 (Thermo Scientific) instrument.

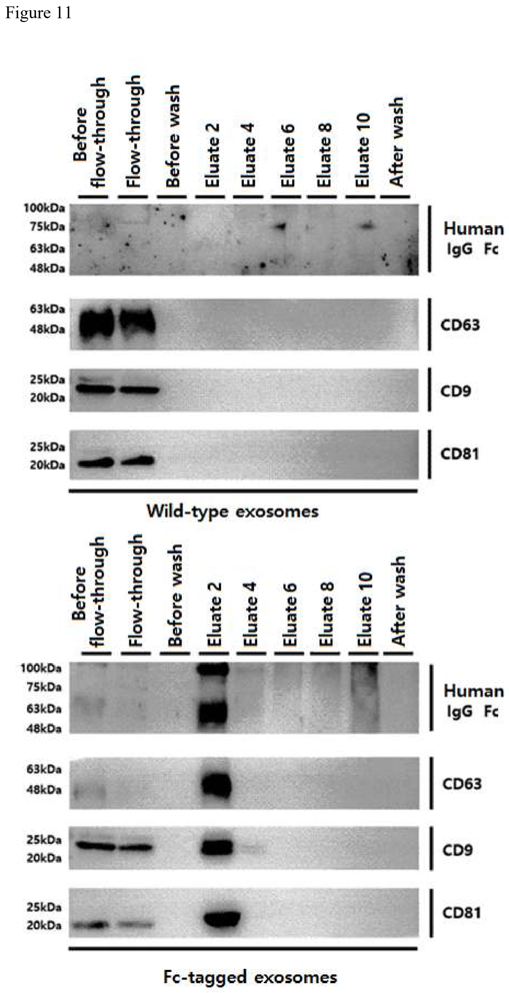

As a result, as shown in Figure 11, it was confirmed that Fc was expressed on the exosome surface. In addition, when comparing the expression levels of representative exosome markers, CD63, CD81, and CD9, in each fraction before and after purification, it was confirmed that exosomes were concentrated only in a specific fraction (Elution 2). The exosomes that do not express the Fc region were removed through flow-through, indicating that this purification method effectively isolated only exosomes comprising Fc.

### Experimental Example 8. Confirmation of purity of isolated exosomes

Each fraction eluate obtained in Example 4 was subjected to electrophoresis and blocking in the same manner as in Experimental Example 7 above. After blocking, the eluate was washed with TBS-T washing buffer. Thereafter, the eluate was sequentially reacted with an antibody specific for the impurity marker, calnexin, and its corresponding secondary antibody. Thereafter, the eluate was reacted with Supersignal West Pico Plus solution (Thermo Scientific, #34580) and analyzed using an iBright 1500 (Thermo Scientific) instrument.

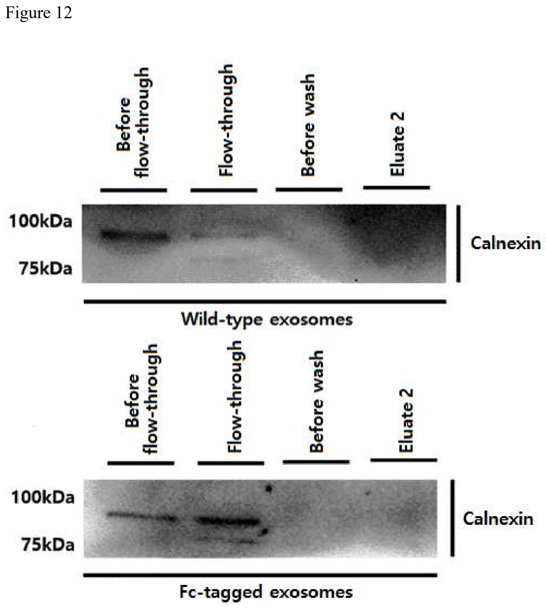

As a result, as shown in Figure 12, the non-exosomal marker, calnexin, was removed by the flow-through and was not present in the exosome-concentrated fraction (Elution 2). Therefore, it can be seen that only exosomes comprising the Fc region are highly concentrated in the Elution 2 fraction after this purification, while the non-exosomal vesicles are excluded.

### Experimental Example 9. Confirmation of Fc expression in each fraction obtained during purification process

The eluates from each fraction obtained during Example 4 were analyzed by flow cytometry using magnetic beads to determine the level of Fc expression on the exosome surface.

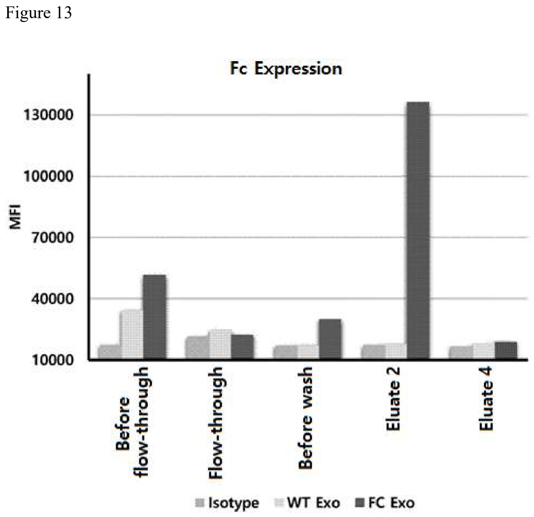

As a result, as shown in Figure 13, it was confirmed that the Fc, which was expressed in small amounts in the IgG Fc KI single cell clone before purification, was concentrated in the Elution 2 fraction during the purification process. Therefore, it was confirmed that the exosomes comprising Fc molecules were purified with high purity through this purification process.

### Experimental Example 10. Confirmation of Fc expression in exosomes obtained during purification process

The Fc expression of the data corresponding to the Elution 2 fraction obtained in Experimental Example 8 was expressed as a histogram.

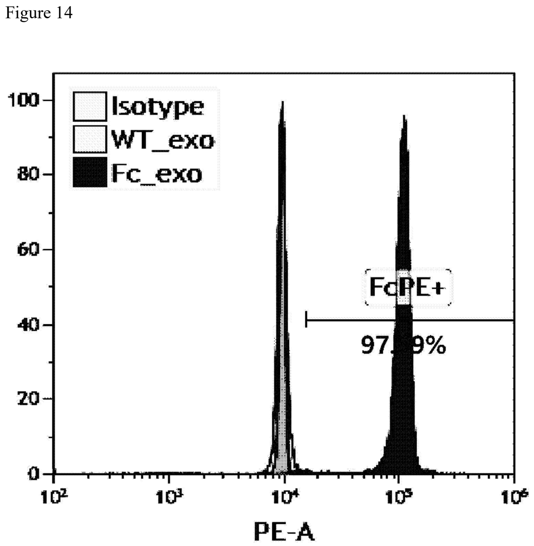

As shown in Figure 14, it was confirmed that Fc was not expressed at all in WT exosomes, but Fc was expressed at a high level at a constant level in exosomes obtained from Fc KI HEK293 single cell clones. In addition, it was confirmed that it was not detected using an isotype control antibody. In summary, these results suggest that the exosomes present in the cell culture solution can be selectively purified with high purity by producing cells that express Fc on the surface of exosomes and passing the cell culture solution obtained during the cell culture process through a chromatography column that selectively binds to IgG Fc and eluting it.

## Claims

1. A fusion protein comprising an extracellular vesicle membrane protein and an immunoglobulin Fc region or a variant thereof.

2. The fusion protein according to claim 1, wherein the Fc is bound to the N-terminus or C-terminus of the extracellular vesicle membrane protein, or is included within the extracellular vesicle membrane protein.

3. The fusion protein according to claim 2, wherein the extracellular vesicle membrane protein is CD63, CD81, CD9, or a combination thereof.

4. The fusion protein according to claim 3, wherein the interior of CD63 is located between the 32nd amino acid residue and the 53rd amino acid residue or between the 105th amino acid residue and the 209th amino acid residue in the amino acid sequence of SEQ ID NO: 1.

5. The fusion protein according to claim 3, wherein the interior of CD81 is located between the 34th amino acid residue and the 63rd amino acid residue or between the 112th amino acid residue and the 201st amino acid residue in the amino acid sequence of SEQ ID NO: 2.

6. The fusion protein according to claim 3, wherein the interior of CD9 is located between the 36th amino acid residue and the 55th amino acid residue or between the 113th amino acid residue and the 196th amino acid residue in the amino acid sequence of SEQ ID NO: 3.

7. The recombinant extracellular vesicle according to claim 1, wherein the Fc region comprises CH2 and CH3 domains.

8. The fusion protein according to claim 1, wherein the Fc variant has a modified CH2 domain.

9. The fusion protein according to claim 1, wherein the Fc is derived from IgG, IgA, IgD, IgM, IgE, or a combination thereof.

10. A polynucleotide encoding the fusion protein according to claim 1.

11. A cell comprising the polynucleotide according to claim 10.

12. The cell according to claim 11, wherein the cell is any one selected from the group consisting of CHO cells, F2N cells, COS cells, BHK cells, Bowes melanoma cells, HeLa cells, 911 cells, AT1080 cells, A549 cells, SP2/0 cells, NSO cells, HT-1080 cells, PERC.6 cells, HEK 293 cells, HEK293T cells, and stem cells.

13. The cell according to claim 12, wherein the stem cell is a mesenchymal stem cell, a hematopoietic stem cell, a neural stem cell, an embryonic stem cell, a pluripotent stem cell, or an induced pluripotent stem cell.

14. A method for producing extracellular vesicles comprising an Fc region or a variant thereof, comprising:
isolating extracellular vesicles from the cell culture solution according to claim 11.

15. A recombinant extracellular vesicle comprising an immunoglobulin Fc region or a variant thereof.

16. The recombinant extracellular vesicle according to claim 15, wherein the Fc region is present on the outer surface of the extracellular vesicle.

17. The recombinant extracellular vesicle according to claim 16, wherein the Fc region is bound to the extracellular vesicle membrane protein.

18. The recombinant extracellular vesicle according to claim 17, wherein the extracellular vesicle membrane protein is any one selected from the group consisting of Alix, Tsg101, Rab protein, ICAM, CD106, CD82, CD58, CD53, CD37, CD13, MFGE8, IGSF8, PTGFRN, MARCKS, MARKCSL1, BASP1, LAMP-1, LAMP-2, CD9, CD81, CD63, and a combination thereof.

19. The recombinant extracellular vesicle according to claim 17, wherein when the extracellular vesicle membrane protein is a peripheral membrane protein, the Fc region is bound to the N-terminus or C-terminus of the extracellular vesicle membrane protein, or is included within the extracellular vesicle membrane protein.

20. The recombinant extracellular vesicle according to claim 17, wherein when the extracellular vesicle membrane protein is an integral membrane protein, the Fc region is bound to at least one selected from the group consisting of the N-terminus, C-terminus, and extracellular domain of the extracellular vesicle membrane protein.

21. The recombinant extracellular vesicle according to claim 15, wherein the Fc region comprises CH2 and CH3 domains.

22. The recombinant extracellular vesicle according to claim 15, wherein the Fc variant has a modified CH2 domain.

23. The recombinant extracellular vesicle according to claim 15, wherein the Fc is derived from IgG, IgA, IgD, IgM, IgE, or a combination thereof.
